# EUROPEAN PATENT APPLICATION

(11) **EP 1 935 337 A1**
(43) Date of publication of application: **25.06.2008**
(21) Application number: 06077297.7
(22) Date of filing: 21.12.2006
(51) Int. Cl.: A61B 5/05

(54) **An electromagnetic imaging system, a method and a computer program product**

(71) Applicant: Nederlandse Organisatie voor toegepast- natuurwetenschappelijk onderzoek TNO, 2628 VK The Hague (NL)
(72) Inventor: Van Ewijk, Lucas Johannes, 3135 JD Vlaardingen (NL)
(74) Representative: van Loon, C.J.J.

(57) **Abstract**

The invention relates to an electromagnetic imaging system for non-invasively imaging an internal structure of a body. The system comprises an external electromagnetic source for generating electromagnetic waves incident upon a measurement location of the body. Further, the system comprises an external receiver for receiving electromagnetic waves propagating from the body after interaction of the incident electromagnetic waves with the measurement location of the body. In addition, the external source is arranged for transmitting non-coherent electromagnetic waves having a relatively large bandwidth.

## Description

The invention relates to an electromagnetic imaging system for non-invasively imaging an internal structure of a body, comprising an external electromagnetic source for generating electromagnetic waves incident upon a measurement location of the body, and an external receiver for receiving electromagnetic waves propagating from the body after interaction of the incident electromagnetic waves with the measurement location of the body.

X-rays using film and other detectors have had medical and industrial application for over one hundred years. Ultra sound has been used for certain medical and industrial applications for about 50 years. Further, computer-aided tomography (CAT) scanning, using ionizing radiation and radioactive tracers, and magnetic resonance imaging (MRI) technology have been used for about 30 years. All of the ionizing radiation systems have dangers and risks associated with their use, in particular to human subjects. MRI systems are less invasive but use a large and very expensive superconducting magnet, making them stationary and expensive to use.

In the use of X-ray an electromagnetic imaging system according to the preamble is known. A X-ray wave generated by a source is directed to a measurement location of a body, interacts with the body, propagates from the body and is received by an external receiver for further processing. The transmission rate of the incident X-ray is a measure for tissue properties.

It is an object of the invention to provide an electromagnetic imaging system according to the preamble, wherein the disadvantages identified above is reduced. In particular, the invention aims at obtaining an electromagnetic imaging system according to the preamble that is less harmful. Thereto, according to the invention, the external source is arranged for transmitting non-coherent electromagnetic waves having a relatively large bandwidth.

By employing non-coherent electromagnetic waves having a relatively large bandwidth, an alternative imaging system is obtained that is practically harmless for persons that are exposed to electromagnetic waves of the system, but also for employers using such a system. Therefore, the system according to the invention is well suited for medical applications, including diagnostics.

Further, the system can be implemented relatively simply as the generation of non-coherent electromagnetic waves can be performed in a simple, cheap way. By using broadband electromagnetic waves, frequency information over a wide frequency band can be obtained for retrieval of electromagnetic information of the measurement location of the body. Further, the system according to the invention might be fast in providing an image of the measurement location to be inspected, optionally in digital format that is fit for further processing, archiving or for remote inspecting. As risk problems of the system are practically zero and a measurement can be accomplished relatively fast, a new measurement can be performed immediately, if desired.

By integrating received electromagnetic wave energy over a frequency range, and allocating the integrated energy to the measurement location of the body, measurement data can be visualized and interpreted simply, without executing complex processing data steps. If desired, however, more dedicated algorithms can be applied to the received data, e.g. for correcting radio wave paths.

The invention also relates to a method of non-invasively imaging an internal structure of a body.

Further, the invention relates to a computer program product.

The invention also relates to a computer system.

Other advantageous embodiments according to the invention are described in the following claims.

By way of example only, embodiments of the present invention will now be described with reference to the accompanying figures in which
Fig. 1 shows a first embodiment of an electromagnetic imaging system according to the invention,
Fig. 2 shows a second embodiment of an electromagnetic imaging system according to the invention,
Fig 3. shows a computer system according to the invention.

It is noted that the figures shows merely preferred embodiments according to the invention. In the figures, the same reference numbers refer to equal or corresponding parts.

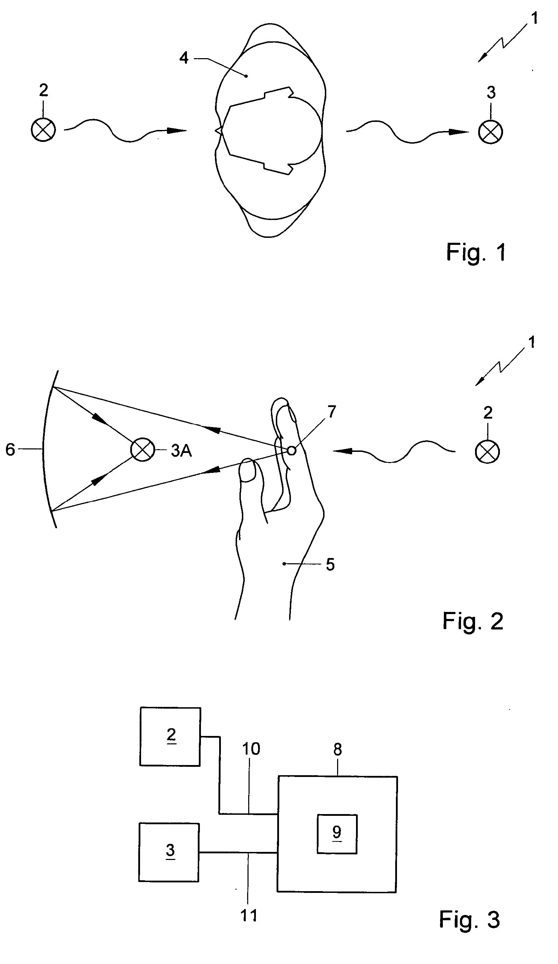

Figure 1 shows a first embodiment of an electromagnetic imaging system 1 according to the invention.

The system 1 is provided with an external electromagnetic source 2 for generating electromagnetic waves incident upon a measurement location of a human body 4, e.g. the thorax of person. Further, the system is provided with an external receiver 3 for receiving electromagnetic waves propagating from the body 4 after interaction of the incident electromagnetic waves with the measurement location of the body.

The external source 2 is arranged for transmitting non-coherent electromagnetic waves having a relatively large bandwidth, preferably substantially larger than half a central frequency of the generated electromagnetic waves, more preferably larger than a central frequency of the generated electromagnetic waves. Obviously, the bandwidth is bounded by a double central frequency of the generated electromagnetic waves, wherein the central frequency of the generated electromagnetic waves is substantially in a range of 1-10 GHz. More preferably, the central frequency is in a range of 1-3 GHz, e.g. 2 GHz. In the latter situation, the bandwidth can amount to 4 GHz. However, the bandwidth can be chosen smaller, e.g. 3 GHz or 2 GHz. Anyhow, the external source 2 generates during operation a broadband non-coherent electromagnetic signal.

After receipt of the electromagnetic waves by the receiver 3, the received electromagnetic wave energy is integrated over a frequency range, and the integrated energy is allocated to the measurement location of the body, so that the measurement data can be visualized and interpreted by users of the system.

In order to perform the integration and allocation steps, the system is provided with a computer system 8 comprising a processor 9 coupled to the external source 2 and to the external receiver 3 via a first and a second input terminal 10, 11, see Figure 3. It is noted that the processing steps mentioned above can be performed either by dedicated hardware or by standard hardware that is loaded with software suitable for performing the data processing tasks.

Advantageously, the external receiver 3 is implemented as a radiometer, so that a relatively cheap receiver is obtained that is arranged to perform relatively accurate measurements over a relatively broad frequency band. It is noted, however, that the receiver can also be implemented otherwise, e.g. by an assembly of receiver elements wherein each of the receiver elements is optimized for receiving signals in a predetermined electromagnetic spectrum.

Further, the external source 2 is preferably a broadband non-coherent noise generator, providing a relatively simple electromagnetic non-coherent source. Of course, various other non-coherent sources could be applied, such as other resistive elements.

Figure 2 shows a second embodiment of an electromagnetic imaging system 1 according to the invention, wherein the receiver 3 comprises an elliptical reflector 6 and an antenna 3A located in a focal point of the reflector 6. By placing the measurement location 7 of the human body, e.g. a hand, in the second focal point of the reflector object, a relatively accurate measurement having a relatively large resolution can be obtained, since waves travelling from the measurement location 7 towards the reflector 6 arrive at the receiver antenna 3A in the first focal point.

In principle, a similar configuration can also be arranged for the source 2, or even in combination with the receiver structure shown in Figure 2 to further enhance the measurement.

The system 1 can be used for visualizing electromagnetic properties of internal human tissue, i.e. electrical conductivity, electric permittivity and magnetic permeability. As a practical applications, the system could be used for medical diagnostic purposes as flesh and fat are more transparent for electromagnetic waves than bone structures. As an example, bone fractures can thus easily be determined. In another application, any presence of drugs in the stomach of a person can be determined.

The invention is not restricted to the embodiments described herein. It will be understood that many variants are possible.

Instead of using the system according to the invention for performing a non-invasive imaging process on human or animal bodies, the system could also be used for other applications, such as in the field of security.

The embodiments described above are of the transmission type. It is noted, however, that the system according to the invention can also be applied for performing a reflection type measurement.

Other such variants will be obvious for the person skilled in the art and are considered to lie within the scope of the invention as formulated in the following claims.

## Claims

1. An electromagnetic imaging system for non-invasively imaging an internal structure of a body, comprising
- an external electromagnetic source for generating electromagnetic waves incident upon a measurement location of the body, and
- an external receiver for receiving electromagnetic waves propagating from the body after interaction of the incident electromagnetic waves with the measurement location of the body,
wherein the external source is arranged for transmitting non-coherent electromagnetic waves having a relatively large bandwidth.

2. An electromagnetic imaging system according to claim 1, wherein the bandwidth substantially equals half of a central frequency of the generated electromagnetic waves.

3. An electromagnetic imaging system according to claim 1 or 2, wherein the central frequency of the generated electromagnetic waves is substantially in a range of 1-10 GHz.

4. An electromagnetic imaging system according to any previous claim, wherein the system is arranged for performing a reflection or a transmission measurement.

5. An electromagnetic imaging system according to any previous claim, wherein the external receiver is implemented as a radiometer.

6. An electromagnetic imaging system according to any previous claim, wherein the external source is a broadband non-coherent noise generator.

7. An electromagnetic imaging system according to any previous claim, wherein the source and/or the receiver comprises an elliptical reflector and an antenna located in a focal point of the reflector.

8. An electromagnetic imaging system according to any previous claim, further comprising a computer system for integrating received electromagnetic wave energy over a frequency range and for allocating the integrated energy to the measurement location of the body.

9. A method of non-invasively imaging an internal structure of a body, comprising the steps of:
- generating electromagnetic waves,
- directing the generated electromagnetic waves to a measurement location of the body with, and
- receiving electromagnetic waves propagating from the body after interaction of the incident waves with the measurement of the body,
wherein the generated electromagnetic waves are non-coherent and have a relatively large bandwidth.

10. A method according to claim 9, further comprising the steps of:
- integrating received electromagnetic wave energy over a frequency range, and
- allocating the integrated energy to the measurement location of the body.

11. A computer program product comprising computer readable code for causing a processor to perform a data processing method for non-invasively imaging an internal structure of a body, comprising the steps of:
- integrating electromagnetic wave energy over a frequency range, the electromagnetic wave energy being received from electromagnetic waves propagating from a measurement location of a body that has been subjected to incident electromagnetic waves, and
- allocating the integrated energy to the measurement location of the body,
wherein the generated electromagnetic waves are non-coherent and have a relatively large bandwidth.

12. Computer system comprising a processor, a first input terminal for connection to an external electromagnetic source for generating electromagnetic waves incident upon a measurement location of the body, and a second input terminal for connection to an external receiver for receiving electromagnetic waves propagating from the body after interaction of the incident electromagnetic waves with the measurement location of the body, wherein the external source is arranged for transmitting non-coherent electromagnetic waves having a relatively large bandwidth, wherein the processor is arranged for integrating received electromagnetic wave energy over a frequency range, and for allocating the integrated energy to the measurement location of the body.
